(19) **Europäisches Patentamt**
**European Patent Office**
**Office européen des brevets**

(11) Veröffentlichungsnummer: **0 285 004 B1**

(12) # EUROPÄISCHE PATENTSCHRIFT

(45) Veröffentlichungstag der Patentschrift:
24.07.91 Patentblatt 91/30

(51) Int. Cl.⁵: **C07C 43/04, C07C 31/04, C07C 41/09, C07C 29/15**

(21) Anmeldenummer: 88104742.7

(22) Anmeldetag: 24.03.88

(54) Verfahren zur Herstellung von Dimethylether.

(30) Priorität: 30.03.87 DE 3710501

(43) Veröffentlichungstag der Anmeldung:
05.10.88 Patentblatt 88/40

(45) Bekanntmachung des Hinweises auf die
Patenterteilung:
24.07.91 Patentblatt 91/30

(84) Benannte Vertragsstaaten:
AT BE CH DE ES FR GB GR IT LI LU NL SE

(56) Entgegenhaltungen:
FR-A- 2 211 437

(73) Patentinhaber: RWE-DEA Aktiengesellschaft
für Mineraloel und Chemie
Überseering 40
W-2000 Hamburg 60 (DE)

(72) Erfinder: Dornhagen, Horst, Dr.
Auf dem Radacker 21
W-5047 Wesseling (DE)
Erfinder: Müller, Manfred, Dr.
Alemannenweg 11
W-5047 Wesseling-Urfeld (DE)

(74) Vertreter: Schupfner, Gerhard D.
Müller, Schupfner & Gauger Karlstrasse 5
Postfach 17 53
W-2110 Buchholz/Nordheide (DE)

## Beschreibung

Die Erfindung betrifft ein Verfahren zur katalytischen Herstellung von Dimethylether aus Methanol in einer apparativen Vorrichtung, die vor einem Reaktor zur Erzeugung von Methanol angeordnet ist.

Methanol wurde während eines Zeitraums von mehr als 40 Jahren ausschließlich durch Hochdruckverfahren erzeugt. Bei diesem Verfahren wird bei Drücken von 250-350 bar und Temperaturen von 320-380°C in Gegenwart von $ZnO/Cr_2O_3$-Katalysatoren aus Synthesegas ($CO/H_2$) sog. Rohmethanol erzeugt, das anschliessend destillativ gereinigt wird.

Bei der Destillation kann man als Nebenprodukt Dimethylether gewinnen. Dieser entsteht im Methanol-Synthesereaktor in einer Menge von bis zu 5 Gew.%.

Seit etwa 1970 wurde das Hochdruckverfahren durch wirtschaftlichere Niederdruckverfahren verdrängt.

In den Niederdruckverfahren werden im allgemeinen Kupfer enthaltende Katalysatoren eingesetzt. Diese erlauben es, die Umsetzung von CO und $H_2$ zu Methanol bei niedrigeren Temperaturen durchzuführen. Hierdurch wird die exotherme Methanolbildung begünstigt,

$$2\,H_2 + CO \rightleftharpoons \overline{CH_3OH}$$

und das Gleichgewicht zum Methanol verschoben. Als Folge kann man auf den hohen Druck der früheren Verfahren verzichten.

Voraussetzung für die Einführung der Niederdruck-Verfahren in die Technik war die Entwicklung von Verfahren zur Reinigung des eingesetzten Synthesegases bis zu einem Schwefelgehalt von ca. 0,1 ppm im Dauerbetrieb. Nur bei diesem hohen Reinheitsgrad sind mit kupferhaltigen Katalysatoren wirtschaftliche Standzeiten erreichbar.

Schon Ende der 50er Jahre gelang es der ICI Synthesegas durch Überleiten über Zinkoxid bis zu der erforderlichen Reinheit zu entschwefeln.

Auch durch Tieftemperaturwäsche mit tiefgekühltem Methanol lässt sich der erforderliche Reinheitsgrad erreichen. Während das ICI-Niederdruck-Methanolverfahren bei etwa 100 bar arbeitet, gelingt es bei dem etwa im gleichen Zeitraum entwickelten Lurgi-Verfahren, die Umsetzung bei 50-60 bar durchzuführen.

Weitere Niederdruckverfahren wurden beispielsweise von Topsoe und Linde entwickelt.

Allen Niederdruckverfahren gemeinsam ist die geringe Bildung von Nebenprodukten, insbesondere auch des Dimethylethers.

Es sind daher mehrere, von der Methanolsynthese unabhängige, katalytische Verfahren zur Synthese von Dimethylether aus Methanol entwickelt worden.

Als technisch wichtigste Katalysatoren haben sich gemäß DE-PS 28 18 831, DE-OS 32 01 155, EP-A-0 099 676 und EP-A-0 124 078 insbesondere Aluminiumoxid- und Aluminiumsilikat-Katalysatoren mit und ohne Dotierung durchgesetzt.

Der hierbei anfallende rohe Dimethylether enthält das Reaktionswasser, nicht umgesetztes Methanol sowie kleine Mengen an Verunreinigungen, wie beispielsweise Methylformiat, Kohlenwasserstoffe, Amine und Sulfide.

In diesen Synthese-Anlagen wird der rohe Dimethylether in zwei hintereinander geschalteten Destillationskolonnen aufgearbeitet, wobei in der ersten, unter Druck betriebenen Kolonne Dimethylether gewonnen wird und in der zweiten nicht umgesetztes Methanol zurückgewonnen wird.

Die Anmelderin der vorliegenden Anmeldung hat ein Verfahren zur Herstellung eines hochreinen Dimethylethers am Deutschen Patentamt unter der Nr. P 36 42 845.0 und ein Verfahren zur Reinigung von Dimethylether am Australischen Patentamt unter der Nr. 65506/86 zum Patent angemeldet.

Die vorliegende Erfindung betrifft ein Verfahren, das für Betreiber von Niederdruck-Methanolanlagen besonders gut geeignet ist, sofern in diesen ein dem Methanolsynthesereaktor vorgeschalteter Entschwefelungsreaktor vorhanden ist. Das erfindungsgemäße Verfahren zur katalytischen Herstellung von Dimethylether aus Methanol ist daher dadurch gekennzeichnet, daß der Dehydratisierungskatalysator in dem (den) Synthesegas-Entschwefelungsreaktor(en), der (die) vor dem Methanol-Synthesereaktor angeordnet ist (sind), enthalten ist.

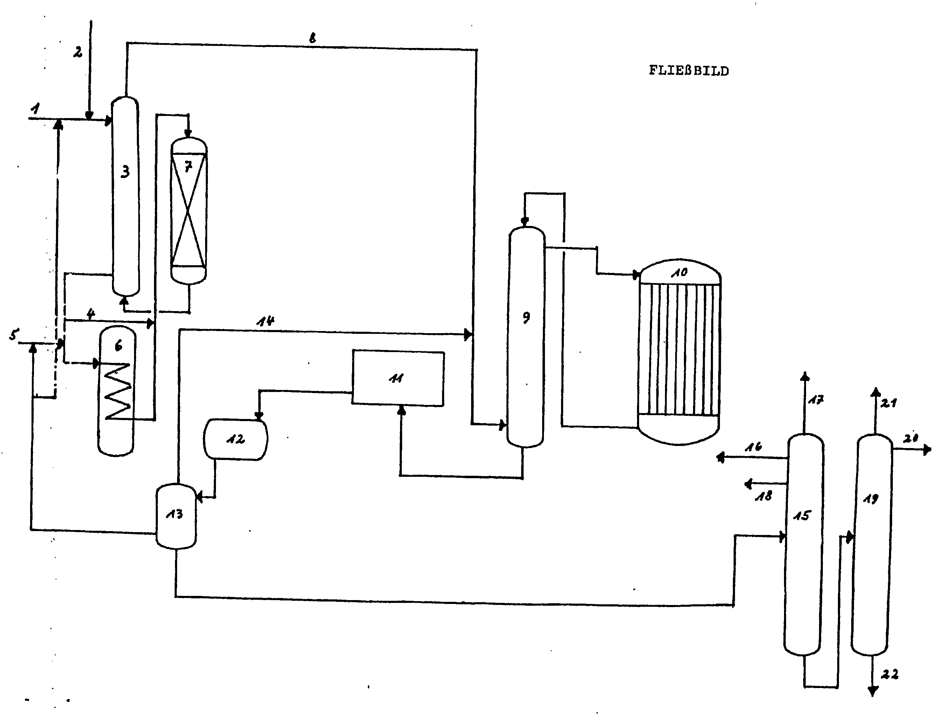

In der Figur 1 ist beispielhaft eine Ausführung des erfindungsgemäßen Verfahrens näher beschrieben.

Entschwefelungsreaktoren zur Reinigung des Synthesegases arbeiten, wie bereits oben ausgeführt, mit ZnO als Entschwefelungsmittel.

Sie werden dann eingesetzt, wenn die Methanolsynthese mit schwefelempfindlichen Katalysatoren durchgeführt wird, insbesondere mit solchen, die Kupfer enthalten, und keine Synthesegaswäsche vorhanden ist, die es erlaubt ebenfalls eine Reinigung des Synthesegases bis zu sehr niedrigen Restschwefelmengen durchzuführen. Beispielhaft sei die Rectisolwäsche mit tiefgekühltem Methanol genannt. Eine vorteilhafte Anordung zur Entschwefelung kann jedoch auch eine Kombination von Wäsche und ZnO-Reaktor sein.

Es sind zahlreiche ZnO-Entschwefelungskatalysatoren bekannt. Sie können außer ZnO noch weitere Komponenten in unterschiedlichen Mengen enthalten.

Beispielhaft seien $Al_2O_3$, $TiO_2$ oder $SiO_2$ genannt. Oft sind jedoch Herstellung und Zusammensetzung der Katalysatoren im einzelnen nicht bekannt.

Obgleich Katalysatoren auf dem Markt erhältlich sind, die bereits bei Raumtemperatur aktiv sind, wird im allgemeinen bei Temperaturen von bis zu 500°C gearbeitet. In Abhängigkeit von der Menge an $CO_2$ im Synthesegas ist es erforderlich bei wenigstens 140°C, besser bei mindestens 180°C zu arbeiten, um die Inaktivierung des Katalysators durch Bildung von $ZnCO_3$ zu vermeiden. Häufig wird bei noch höheren Temperaturen, bevorzugt im Temperaturbereich von 180°C bis 480°C, meist bevorzugt von 250 bis 440°C gearbeitet.

Erfindungsgemäß wird im Entschwefelungsreaktor auch der Dehydratisierungskatalysator zur Gewinnung von Dimethylether aus Methanol angeordnet, wobei in den Reaktor neben dem zu entschwefelnden Synthesegas, dampfförmiges Methanol in der erforderlichen Menge zugeführt wird. Methanol kann hierzu durch Verdampfen flüssigen Methanols erhalten werden, es kann jedoch auch hinter dem Methanol-Synthesereaktor dampfförmig entnommen werden, bevor es durch Kühlen verflüssigt worden ist. Der erzeugte Dimethylether durchströmt den Methanol-Synthesereaktor und wird in der Methanolaufarbeitung destillativ abgetrennt und gereinigt.

Der Dehydratisierungskatalysator kann mit dem Entschwefelungskatalysator gemischt sein, er kann jedoch auch in getrennten Schichten angeordnet sein, beispielsweise in einer dem Entschwefelungskatalysator nach- oder vorgeschalteten Schicht oder sowohl einer vor-, als auch nachgeschalteten Schicht. Auch mehrere Schichten beider Katalysatoren können erfindungsgemäß vorliegen.

Obgleich erfindungsgemäß zahlreiche Dehydratisierungskatalysatoren verwendet werden können, werden $Al_2O_3$— und Aluminiumsilikat-Katalysatoren bevorzugt, hierzu gehören natürliche und künstliche $Al_2O_3$— und Aluminiumsilikat-Katalysatoren wie z.B. Zeolithe.

Besonders geeignete Katalysatoren sind beispielsweise $\gamma$-$Al_2O_3$-Katalysatoren, insbesondere auch solche mit niedrigem $SiO_2$-Gehalt (z.B. einem $\gamma$-$Al_2O_3$-katalysator der von 0,0001 bis < 1 Gew.% $SiO_2$ enthält), wie sie in der deutschen Patentanmeldung P 36 42 845.0 offenbart sind.

Geeignet sind auch Entschwefelungs-Katalysatoren, die Anteile an $Al_2O_3$ und/oder Aluminiumsilikate enthalten. Hierzu können gezielt solche Mengen an diesen Komponenten enthalten sein, daß Dimethylether in der gewünschten Menge gebildet wird.

Durch die Nutzung des Entschwefelungsreaktors zur gleichzeitigen Erzeugung von Dimethylether ist eine besonders wirtschaftliche Erzeugung des Dimethylethers möglich, da zusätzliche Investitionen für eine getrennte Dimethylether-Synthese nicht erforderlich sind und das den Dimethylether enthaltende Rohmethanol in der destillativen Rohmethanolaufarbeitung in hoher Reinheit gewonnen werden kann. Dies ist insbesondere durch eine Fahrweise möglich, bei der man den Dimethylether in einer Kolonne als Reinprodukt seitlich wenige Böden unter dem Kopf der Kolonne abzieht, wobei gleichzeitig im unteren Teil der Kolonne durch Rückfluß des im Überschuß eingesetzten Methanols eine besonders wirkungsvolle Reklifikation erfolgt.

Die vorliegende Erfindung wird an Hand der Figur näher erläutert.

Synthesegas fließt über (1) in Wärmetauscher (3). Über (2) wird Methanol zugeführt. Das Gemisch fließt von (3) über (4) bzw. über Überhitzer (6), soweit das zur Aufrechterhaltung der Temperatur erforderlich ist, in Entschwefelungsreaktor (7). Methanol kann beispielsweise auch über (5) zugeführt werden.

Das entschwefelte Gemisch gelangt über (8) in Wärmetauscher (9) und von dort in Methanolreaktor (10). Das Dimethylether enthaltende Rohmethanol gelangt über (9), Luftkühler (11) und Wasserkühler (12) in Abscheider (13). Kreislaufgas fließt über (14) nach (9).

Das Rohmethanol gelangt in Kolonne (15), wo über (16) reiner Dimethylether abgezogen wird. Verunreinigungen können über (17) und/oder (18) und-/oder (21) entnommen werden.

Der im wesentlichen Methanol, Wasser und höhere Alkohole enthaltende Sumpf aus (15) gelangt in Kolonne (19), wo bei (20) Reinmethanol abgezogen wird, während Wasser und höhere Alkohole zur weiteren Trennung in eine dritte Kolonne geleitet werden können.

Das Fließbild ist als beispielhaft anzusehen. Auch andere Fahrweisen liegen im Rahmen der vorliegenden Erfindung.

Je nach den Fahrbedingungen, insbesondere bei geeignetem Druck in Methanol- und Entschwefelungsreaktor kann beispielsweise auch aus dem Methanolreaktor abgezogenes dampfförmiges Methanol zusammen mit Kreislaufgas direkt in Entschwefelungsreaktor (7) eingesetzt werden.

Die vorliegende Erfindung erlaubt es, die Produktion von Dimethylether und Methanol dem Bedarf flexibel anzupassen. In (13) anfallendes Methanol enthaltendes Kondensat kann beispielsweise dazu teilweise verwendet werden, über (1) oder (2) dem Entschwefelungsreaktor (7) die gewünschte Methanolmenge zuzuführen. Methanol kann auch bei (20) entnommen werden und (7) zugeführt werden.

**Patentansprüche**

1. Verfahren zur katalytischen Herstellung von Dimethylether aus Methanol, dadurch gekennzeichnet, daß der Dehydratisierungs-Katalysator in dem (den) Synthesegas-Entschwefelungsreaktor(en), der (die) vor dem Methanol-Synthesereaktor angeordnet ist (sind), enthalten ist.

2. Verfahren nach Anspruch 1, dadurch gekennzeichnet, daß im Entschwefelungsreaktor Entschwefelungskatalysator und Dehydratisierungs-Katalysator schichtweise angeordnet sind.

3. Verfahren nach den Ansprüchen 1 und 2, dadurch gekennzeichnet, daß der Entschwefelungsreaktor bei einer Temperatur von 140 bis 500°C, vorzugsweise von 180 bis 480°C und besonders bevorzugt von 250 bis 440°C betrieben wird.

4. Verfahren nach den Ansprüchen 1-3, dadurch gekennzeichnet, daß der Dehydratisierungskatalysator dem Entschwefelungskatalysator nachgeschaltet ist.

5. Verfahren nach den Ansprüchen 1-4, dadurch gekennzeichnet, daß der Dehydratisierungskatalysator überwiegend $\gamma$—$Al_2O_3$ enthält.

6. Verfahren nach den Ansprüchen 1-5, dadurch gekennzeichnet, daß über einem $\gamma$—$Al_2O_3$-Katalysator dehydratisiert wird, der von 0,0001 bis < 1 Gew.% $SiO_2$ enthält.

7. Verfahren nach den Ansprüchen 1-6, dadurch gekennzeichnet, daß reiner Dimethylether in einer Destillationskolonne am Kopf oder Seitenabzug abgezogen wird und an einem tieferen Boden Verunreinigungen abgezogen werden, die zwischen Dimethylether und Methanol sieden.


## Claims

1. A process for the catalytic production of dimethyl ether from methanol wherein the synthesis gas desulphurizing reactor(s) placed before the methanol synthesis reactor hold(s) the dehydration catalyst.

2. A process in accordance with claim 1 wherein the desulphurizing catalyst and the dehydration catalyst are arranged in layers in the desulphurizing reactor.

3. A process in accordance with claim 1 and 2 wherein the desulphurizing reactor is operated at a temperature in the range of between 140 and 500°C, preferably between 180 and 480°C, most preferably between 250 and 440°C.

4. A process as claimed in any of the claims 1 to 3 wherein the dehydration catalyst is placed behind the desulphurizing catalyst.

5. A process as claimed in any of the claims 1 to 4 wherein the dehydration catalyst mainly contains gamma-$Al_2O_3$.

6. A process as claimed in any of the claims 1 to 5 wherein a gamma-$Al_2O_3$ catalyst containing 0.0001 to less than 1 wt.% $SiO_2$ is used for the dehydration.

7. A process as claimed in any of the claims 1 to 6 wherein pure dimethyl ether is withdrawn at the top or at the side of a distillation column and those impurities boiling between dimethyl ether and methanol are removed from a lower plate.


## Revendications

1. Procédé pour la fabrication catalytique de l'éther diméthylique à partir du méthanol, caractérisé en ce que le catalyseur de déshydratation est contenu dans le(s) réacteur(s) de désulfuration du gaz de synthèse, placé(s) avant le réacteur de synthèse du méthanol.

2. Procédé selon la revendication 1, caractérisé en ce que le catalyseur de désulfuration et le catalyseur de déshydratation sont placés en couches dans le réacteur de désulfuration.

3. Procédé selon les revendications 1 et 2, caractérisé en ce que le réacteur de désulfuration fonctionne à une température de 140 à 500°C, de préférence de 180 à 480°C et en particulier de 250 à 440°C.

4. Procédé selon les revendications 1-3, caractérisé en ce que le catalyseur de déshydratation est placé après le catalyseur de désulfuration.

5. Procédé selon les revendications 1-4, caractérisé en ce que le catalyseur de déshydratation contient essentiellement $Al_2O_3$ $\gamma$.

6. Procédé selon les revendications 1-5, caractérisé en ce que l'on effectue la déshydratation sur un catalyseur d'$Al_2O_3$ $\gamma$ qui contient de 0,0001 à < 1% en poids de $SiO_2$.

7. Procédé selon les revendications 1-6, caractérisé en ce que l'on retire l'éther diméthylique pur en tête ou par une conduite latérale d'une colonne de distillation et on retire d'un plateau inférieur les impuretés qui bouillent entre l'éther diméthylique et le méthanol.

FLIEßBILD